# EUROPEAN PATENT APPLICATION

(11) **EP 1 053 749 A1**
(43) Date of publication of application: **22.11.2000**
(21) Application number: 99900166.2
(22) Date of filing: 11.01.1999
(51) Int. Cl.: A61K 31/665

(54) **PREVENTIVES AND REMEDIES FOR NEURON DIGENERATION-ASSOCIATED DISEASEAS**

(30) Priority: 03.02.1998 JP 3676098; 19.06.1998 JP 18964598
(71) Applicant: Senju Pharmaceutical Co., Ltd., Osaka-shi, Osaka 541-0046 (JP)
(72) Inventor: MORI, Akitane, Okayama-shi, Okayama 703-8282 (JP); XIN, Wenjuan, Institute of Biophysics, Chaoyang District, Beijing 100101 (CN)
(74) Representative: Behnisch, Werner, Dr.
(86) International application number: JP9900060
(87) International publication number: WO9939716

(57) **Abstract**

Described are medicaments for prevention and treatment of neurodegenerative diseases including Alzheimer's disease, Parkinson's disease and amyotrophic lateral sclerosis, which medicaments comprise a compound represented by the formula, 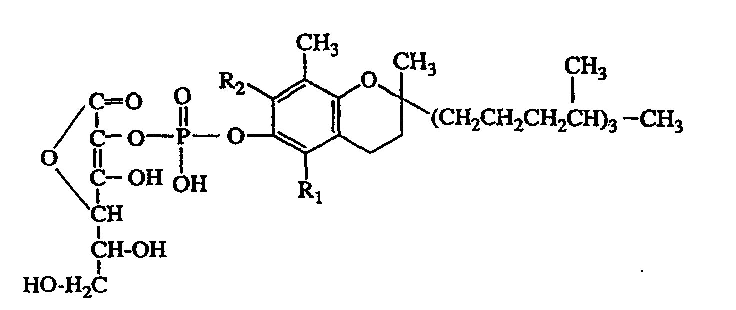 wherein R1 and R2 are the sane or different and each denotes hydrogen or methyl, or a pharmaceutically acceptable salt thereof.

## Description

### Technical Field

The present invention relates to medicaments for prevention of neurodegenerative diseases. More specifically, the present invention relates to medicaments for prevention or treatment of a variety of diseases accompanied by neuronal apoptosis, such as Alzheimer's disease, Parkinson's disease, amyotrophic lateral sclerosis and the like.

### Background Art

Two types of cell deaths, apoptosis and necrosis, may take place under certain physiological and pathological conditions. Apoptosis, also termed as the programmed cell death, is a kind of "self"-regulated cell death which can be triggered by a variety of extrinsic and intrinsic signals. In contrast to necrosis, which is caused by an acute cellular injury and typified by rapid cell swelling and lysis, apoptosis is morphologically characterized by cytoskelton disruption, cell shrinkage, membrane blebbing, and nuclei fragmentation. In the last decade, the role of apoptosis in the pathogenesis of many of neuronal diseases has widely drawn attention [Bredesen, D.E. (1995), Ann. Neurol., 38, 839-851]. Such disorders include Alzheimer's disease, Parkinson's disease, amyotrophic lateral sclerosis, and various forms of cerebellar degeneration [Thompson, C.B. (1995), Science 267, 1456-1462], while more and more evidence has suggested that reactive oxygen species (ROS) might play important roles in the cascade of events leading to neuronal apoptosis [Greenlund, L.J.S., Deckwerth, T.L., and Johnson Jr., E.M., (1995), Neuron 14, 303-315].

On the other hand, nitric oxide (NO), an endogenously generated short-life free radical, participates in a number of functions including neurotransmission, synaptic plasticity and long-term memory in the central nervous system [Garthwaite, J. and Boulton, C.L., (1995), Annu. Rev. Physiol., 57, 683-706]. It has also been reported that NO shows neurotoxicity when produced in large excess or concurrently with ROS, and that NO acts as a pathological mediator in some neurodegenerative diseases [Gross, S.S., and Wolin, M.S., (1995), Annu. Rev. Physiol., 57, 737-769; Zhang, J., and Snyder, S.H., (1995), Annu. Rev. Pharmacol. Toxicol., 35, 213-233]. It has also been reported that NO could induce cell death in cortical neurons [Palluy, O., and Rigaud, M.,(1996), Neurosci. Lett., 208, 1-4], mesencephalic neurons [Grabson-Frodl, E.M., and Brundin, P., (1997), Exp. Brain Res., 113, 138-143] and cerebellar neurons [Bonfoco, E., Leist, M., Zhivotovsky, B., Orrenius, S., Lipton, S.A., and Nicotera, P., (1996), J. Neurochem., 67, 2484-2493]. The corresponding mechanisms, however, are still not known.

Very serious diseases as they are, the mechanisms of those various diseases accompanied by neuronal degeneration and cell deaths have not been elucidated to such an extent that help discover an effective method for their prevention or treatment. Thus, clarification of fundamental mechanisms of those diseases has been needed, together with an early establishment of an effective method for their prevention and treatment. While they progress either gradually or rapidly, prevention of those diseases becomes available if it is possible to block the onset of neuronal degeneration. Even after the neuronal degeneration has started, blocking or slowing down further progress of degeneration, or prevention or inhibition of the degeneration area from expanding, if possible, could make available a method for treatment to substantially control those neuronal diseases.

The compound represented by the following formula, which consists of α -tocopherol or its analogue and L-ascorbic acid linked together via phosphate ester bonds, 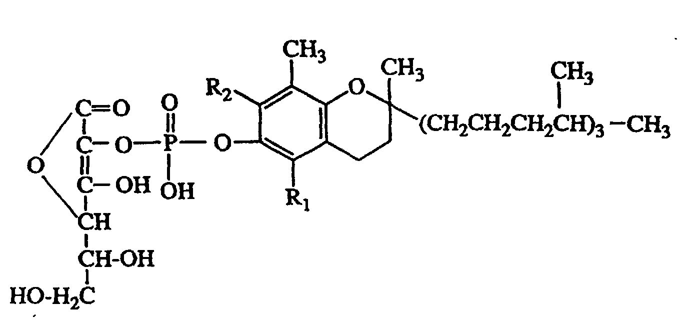 wherein R1 and R2 are the same or different and each denotes hydrogen or methyl (hereinafter referred to as "the present compound") includes a group of compounds that have been known to act as potent antioxidants [Kuribayashi, Y., Naritomi, H., Sasaki, M., and Sawada,T., (1994), Arzneim.-Forsch./Drug Res., 44(II), 995-998; Block, F., Kunkel, M,and Sontag, K-H.,(1995), Brain Res. Bull., 36, 257-260], for which a number of uses are known, such as anticataract agents, agents for prevention and treatment of climacteric disturbance, agents for cosmetics with skin-beautifying effect [Japanese Patent Publication No. H02-44478], anti-inflammatory agents [Japanese Patent Publication No. H01-27004], anti-ulcer agents [Japanese Laid-open Patent Application No. S63-27062], agents for prevention and treatment of ischemic disorders of organs [Japanese Laid-open Patent Application No. H02-111722]. It is not known, however, about what action the present compound could have on the neuronal toxicity of nitrogen oxide, nor has it been investigated whether it can block or suppress apoptosis of neurons.

The objective of the present invention is to provide medicaments for prevention and treatment of those various diseases accompanied by neuronal apoptosis. In the present description, the term "treatment" used is meant to include blocking and suppression of the progression of those diseases.

### Disclosure of Invention

In order to investigate possible pathways involved in NO-induced neuronal damage, the present inventors used primary cultures of rat cerebellar granule cells, which gives a relatively homogeneous population of neurons. In the investigation, the present inventors found for the first time that the present compound that was employed in the analysis of mechanisms has a preventive or suppressive effect on neuronal apoptosis. The present invention was made through further studies based on this finding.

Thus, the present invention provides a medicament for prevention and treatment of neurodegenerative diseases comprising a compound represented by the formula, 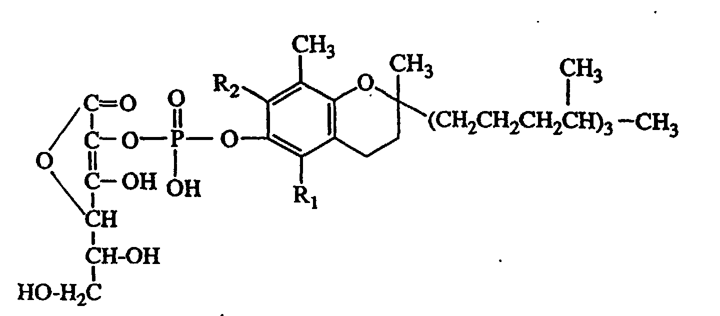 wherein R1 and R2 are the same or different and each denotes hydrogen or methyl, or a pharmaceutically acceptable salt thereof.

Representative examples of the neurodegenerative diseases to which the medicament of the present invention is addressed include Alzheimer's disease, Parkinson's disease and amyotrophic lateral sclerosis. Diseases to be treated by the present invention, however, are not limited to them but also include a variety of neurodegenerative diseases accompanied by neuronal apoptosis.

Therefore, the present invention further provides a method for prevention and treatment of the above neurodegenerative diseases in a mammal including a human comprising administering to the mammal including a human an effective amount of the above compound or a pharmaceutically acceptable salt thereof.

The present invention further provides use of the above compound or a pharmaceutically acceptable salt thereof for preparation of a medicament for prevention and treatment of the above neurodegenerative diseases in a mammal including a human.

### Brief Description of Drawings

Figure 1 is a graph illustrating the change in cell viability induced by exposure to NO donors.
Figure 2 is a graph illustrating the protective effect of radical scavengers against cell death induced by exposure to NO donors.
Figure 3 is a graph illustrating the suppressive effect of radical scavengers against lowering of mitochondrial enzyme activities induced after exposure to GSNO.
Figure 4 is a graph illustrating the preventive effect of radical scavengers against lipid peroxidation.
Figure 5 is a graph illustrating the preventive effect of EPC-K1 against lowering of mitochondrial enzyme activities and cell death induced by peroxynitrite.
Figure 6 is a graph illustrating the change in cell viability induced by exposure to peroxynitrite.
Figure 7 is a graph illustrating the preventive effect against lipid peroxidation.
Figure 8 is a graph illustrating the alteration of cell membrane fluidity.
Figure 9 is a graph illustrating the change in ATP content.

### Best Mode for Carrying Out the Invention

The present compound can be synthesized according to the method described in Japanese Patent Publication Nos. H02-44478 or H05-23274, for example, or analogously thereto.

For the purpose of the present invention, the present compound may be used in its free form or in the form of a pharmaceutically acceptable salt thereof. Representative examples of such salts include, but are not limited to, alkaline metal salts such as sodium salt, potassium salt and the like, and alkaline earth metal salts such as calcium salt, magnesium salt and the like. Other salts may be chosen insofar as they are pharmaceutically acceptable.

The medicament for prevention and treatment of neurodegenerative diseases may contain two or more species of the present compound, which differ in attached radicals, in combination, and may likewise contain two or more salts in combination.

Its very low toxicity gives the present compound a high safety. For example, a potassium salt of phosphodiester of L-ascorbic acid and DL- α - tocopherol (hereinafter referred to as "EPC-K1"), a specific compound included in the present compound, has LD50 values of over 5 g/kg (rat, p.o.) and over 100 mg/kg (rat, i.v.).

The medicament for prevention and treatment of neurodegenerative diseases of the present invention may be administered either orally or parenterally. Thus, as for pharmaceutical preparation forms, it may be either in solid preparations for oral administration including tablets, granules, powders and capsules or in liquid preparations including injections. In the case of an injection, it may be a preparation, which is to be made into a solution prior to use, consisting of the present compound-containing aseptic dry powder prepared by lyophilization, for example, and an aseptic solvent. Any of those preparation forms may be made by a conventional method using the present compound as the active component. Such preparation forms may contain conventionally employed additives such as excipients, binders, thickeners, dispersing agents, resorption enhancers, buffering agents, surfactants, solubilizers, preservatives, emulsifiers, isotonizers, stabilizers, pH adjusting agents and the like.

The dose of the present compound used for prevention and treatment of neurodegenerative diseases may be; e.g., about 1 mg to about 100 mg once a day for an adult, in the case of injections, and about 10 mg to about 1,000 mg at a time, which is repeated several times a day, for an adult, in the case of oral preparations, although the doses may be properly decided by the physician in charge according to the disease to be treated, the body weight and age of the patient, the severity of the disease, and the like.

Through the pharmacological studies that will be described below, the present inventors found that NO donors and peroxynitrite both can induce apoptosis in immature cultures of cerebellar granule cells, and that free radical scavengers can inhibit the process to some extent. The results have further implication for deeper understanding of apoptosis and neurodegenerative processes, which would lead to their selective control.

Thus, the present inventors demonstrated that exposure to 100-250 µ mol/L NO donors S-nitrosoglutathione (GSNO) or sodium nitroprusside (SNP) triggered apoptosis in immature cultures of cerebellar granule cells, and, using this as a model, carried out a series of experiments to make it clear whether reactive oxygen species (ROS) were involved in the NO-associated neuronal damages. It was found that NO-induced cell death could be attenuated by pre-treating the cells with antioxidants EPC-K1 (described hereinafter), which is the active principle contained in the present invention, or SOD (superoxide dismutase). This indicates that ROS is formed while the cells are treated with NO donors. The mechanism may be that the exposure of cells to NO donors causes mitochondrial dysfunction, which leads to the formation of ROS such as superoxide and peroxynitrite, which then causes oxidative injury, finally resulting in apoptosis. The results of the present investigation suggest that ROS-mediated (especially, peroxynitrite-mediated) oxidative stress is an important pathway leading to NO-associated neuronal damage. Pre-treatment of the cells with free radical scavengers EPC-K1 or SOD, attenuated NO-induced apoptosis by inhibiting the formation of peroxynitrite, and by scavenging peroxynitrite and/or its breakdown products.

### 〈Pharmacological Test 1〉

In an experiment of NO-induced neuronal degeneration, one of the present compound, L-ascorbic acid 2-[3,4-dihydro-2,5,7,8-tetramethyl-2-(4,8,12-trimethyltridecyl)-2H-1-benzopyran-6-yl-hydrogen phosphate] phosphate salt, which is the mono-potassium salt of the compound consisting of L-ascorbic acid and α-tocopherol linked together via a phosphate diester (herein referred to "EPC-K1") was used, together with SOD and Hb, as a means for probing the mechanism of neuronal degeneration.

### A. Materials and Methods

(1) Materials: Wistar rats were purchased from Beijing Medical University, China. Cell culture dishes were purchased from Nunc (Denmark). Agarose, deoxyribonuclease I (DNase I), Dulbecco's modified Eagle medium (DMEM), Earle's balanced salt solution (EBSS), proteinase K, trypan blue, and trypsin (1:250) were products of Gibco BRL (U.S.A.). Bovine hemoglobin (Hb), bovine erythrocyte superoxide dismutase (SOD), 3-[4,5-dimethylthiazol-2-yl]-2,5-diphenyl tetrazolium bromide (MTT), ethidium bromide, glutathione (GSH), Hoechst 33258, poly-L-lysine, ribonuclease A (RNase A), and sodium nitroprusside (SNP) were purchased from Sigma (U.S.A.). EPC-K1 used was synthesized in Senju Pharmaceutical Co., Ltd. S-nitrosoglutathione (GSNO) was prepared by a conventional method [Hart, T.W., (1985), Tetrahedron Lett., 26, 2013-2016]. Peroxynitrite was synthesized in a quenched flow reactor [Zhao, B.L., Shen, J.G., Li, M., Li, M.F., Wan, Q., and Xin, W.J., (1996), Biochim. Biophys. Acta, 1315, 131-137] and was diluted with 1 mmol/L NaOH just before use. Other reagents made in China were of analytical grade.
(2) Cell Culture: Primary cultures of rat cerebellar granule cells were prepared following procedures previously reported [Ni, Y.C., Zhao, B.L., Hou, J.W., and Xin, W.J., (1996), Neurosci. Lett., 214, 115-118]. Briefly, cerebella of 7-day-old Wistar rats were collected, rinsed with HBSS, and dissociated by mild trypsinization. The cells were plated in 6-well multidishes (2 × 10⁶ cells/mL, 2 mL/ well) or 24-well multidishes (2.5× 10⁶ cells/mL, 0.4 mL/well) previously coated with poly-L-lysine. The culture medium was DMEM supplemented with KCl (19.6 mmol/L), glutamine (2 mmol/L), HEPES (10 mmol/L) and fetal calf serum (10 %, v/v). The cells were maintained at 37°C in a humidified 5 % CO₂-95 % air atmosphere. Experiments were carried out 48 hrs after the plating.
(3) Exposure to NO: NO donors (GSNO and SNP) from freshly prepared stock solutions were added to the wells and the cells were cultured for indicated length of time. Decomposed NO donors were prepared by incubating the stock solutions at 37°C for 24 hrs. Antioxidants (EPC-K1, SOD, Hb), when employed, were added to the cells 15 min before the addition of NO donors. Positive control was prepared by treating cells with hydroxyl radicals [Ni, Y.C., Zhao, B.L., Hou, J.W. and Xin, W.J., (1996), Neurosci. Lett, 214, 115-118].
(4) Exposure to peroxynitrite: The culture media were carefully replaced with EBSS supplemented with 25 mmol/L HEPES (pH 7.4) and the cells were incubated at 37°C for 15 min. Antioxidants, when employed, were added during this period. Aliquots of the peroxynitrite stock solution were added to the cells, which then were incubated at 37°C for 30 min. To the control cells were added the same volume of 1 mmol/L NaOH. The cells then were rinsed twice with EBSS, the original culture media were restored, and cells were cultured for indicated length of time. Under these experimental conditions, the addition of peroxynitrite only caused slight pH shifts.
(5) Morphological Studies: Morphological features of the cells were observed using both a phase-contrast microscope and a fluoromicroscope. Ultrastructure of the cells was observed by an electron microscope. For fluoromicroscopic observation, cells plated on glass coverslips were fixed with 4 % paraformaldehyde for 25 min, rinsed twice with PBS, stained with Hoechst 33258 (10 µg/mL) for 15 min, and observed with a Nikon Diaphot 300 microscope. Samples for electron microscopy were prepared following a standard protocol. Briefly, cell were fixed with 2.5 % glutaraldehyde at 4°C for 1 hr and post-fixed with 1 % OsO₄ at 4°C for 1 hr, dehydrated through a series of graded ethanol solutions, and embedded in resin. Ultrathin sections of the samples were stained with uranyl acetate and lead citrate and observed using an electron microscope.
(6) DNA Fragmentation Analysis: 4 × 10⁶ cells were collected, rinsed with cold PBS, and lysed in lysis buffer (50 mmol/L Tris, 10 mmol/L EDTA, 0.5 % N-lauroylsarcosine sodium salt, 0.5 mg/mL proteinase K, pH 8.0) by incubating at 50°C for 3 hrs. After phenol and then chloroform extractions, DNA was precipitated with 0.1 volume of 10 mol/L ammonium acetate and 2 volumes of ethanol at -20°C overnight, and harvested by centrifugation at 12,000×g for 15 min. After washed with 70 % ethanol, the DNA was dissolved in TE (10 mmol/L Tris, 1 mmol/L EDTA, pH 8.0), treated with 0.5 mg/mL ribonuclease A at 37°C for 30 min, and electrophoresed in 1 % agarose gel using 0.5 ×TBE (45 mmol/L Tris, 45 mmol/L boric acid, 1 mmol/L EDTA, pH 8.0) as the running buffer. After ethidium bromide staining (0.5 µ g/mL), photographs were taken under UV transillumination.
(7) Determination of Cell Viability: Cell viability was assessed by trypan blue exclusion. After stained with 0.4 % trypan blue, dead and viable cells were counted in 3 random fields per well using an inverted phase-contrast microscope.
(8) MTT Assay: MTT was added to the cells cultured in 24-well multidishes (0.5 mg/mL, final concentration). After incubation at 37°C for 30 min, 1 mL of lysis solution (10 % SDS, 25 % DMF, pH 3.5) was added and optical density at 570 nm was measured.
(9) Determination of Lipid Peroxidation: Thiobarbituric acid (TBA) assay was employed as the measure of lipid peroxidation. 1.2 × 10⁷ cells were pelletted, washed twice, and resuspended in 0.4 mL of PBS. The suspension was mixed with 0.4 mL of 10 % trichloroacetic acid and 1 mL of 0.67 % thiobarbituric acid, and the mixtures were heated at 95°C for 1 hr. After extraction with butanol, thiobarbituric acid-reactive substances (TBARS) were determined at 532 nm on a spectrophotometer.
(10) Statistical Analyses: Statistical analyses were performed using one-way ANOVA or Student's t-test. A probability of <0.05 was deemed significant.

### B. Test Results:

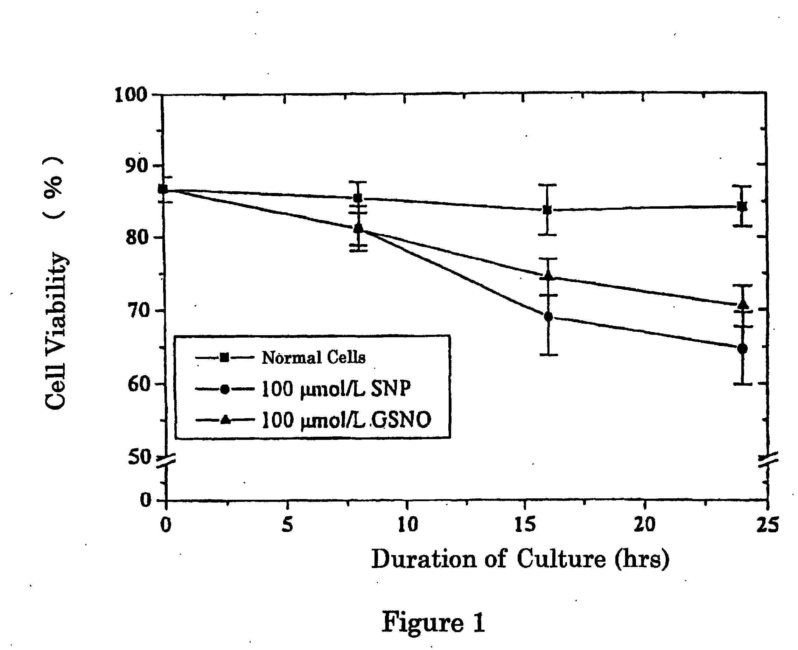

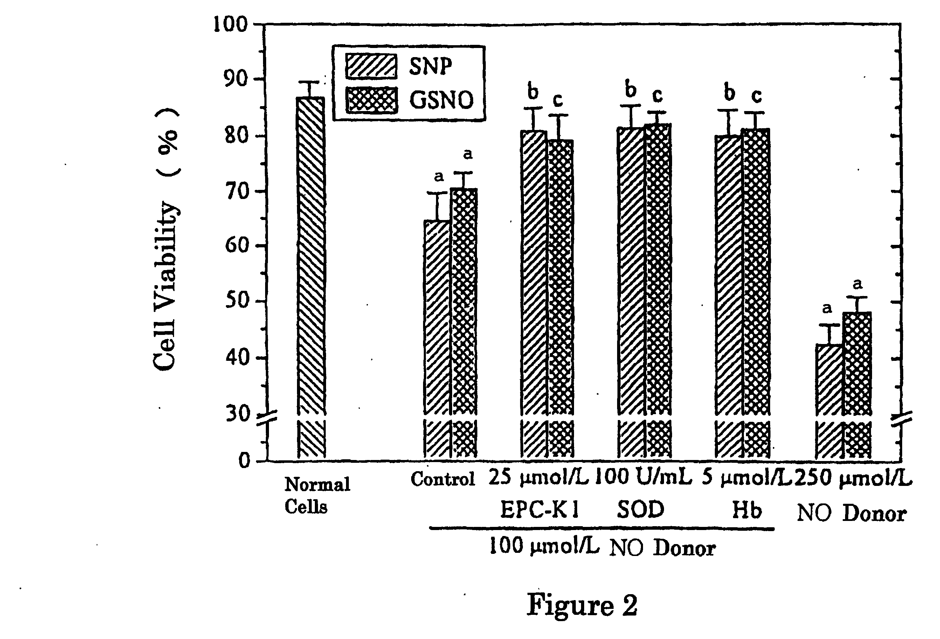

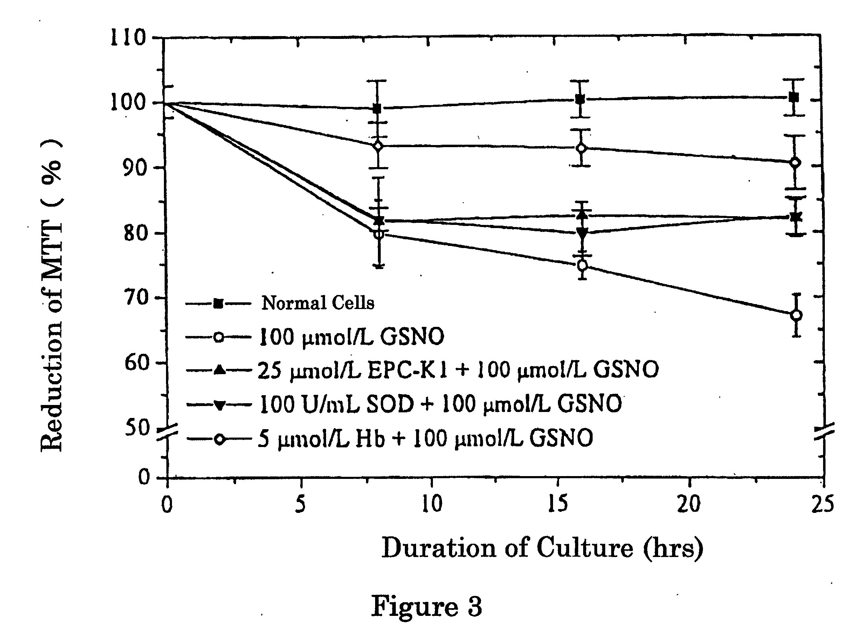

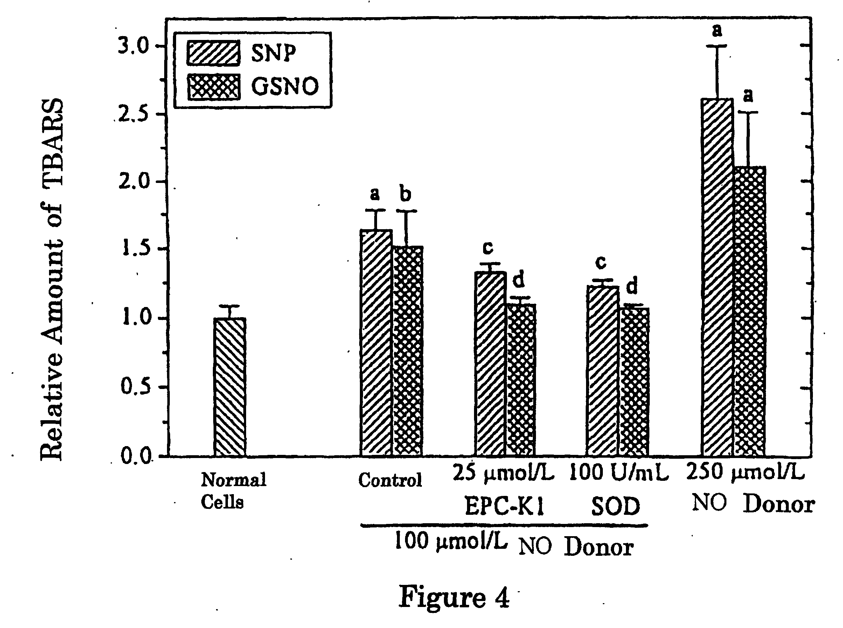

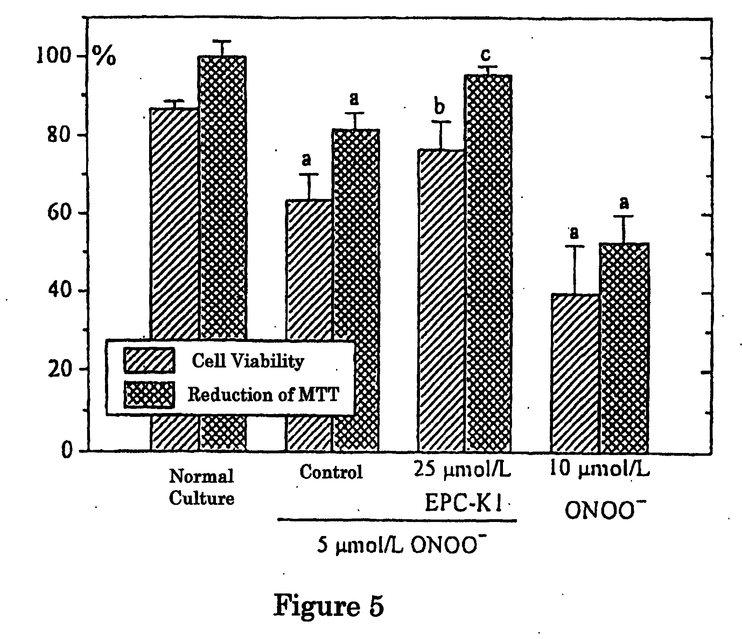

(1) Characteristics of Apoptosis: After the 24-hour exposure to NO donors GSNO or SNP (100-250 µmol/L), the cerebellar granule cells underwent apoptotic cell death, which was characterized by decrease of adherent cells, neurite breakdown, cell shrinkage, chromatin condensation and nuclei fragmentation. Either free radical scavengers, 25 µmol/L EPC-K1 or 100 µmol/L SOD, or a NO scavenger hemoglobin (Hb) protected the cells from death. Agarose gel electrophoresis of DNA extracted from the NO-treated cells showed a ladder pattern, which is a well accepted characteristic of apoptosis. Pre-treatment of the cells with EPC-K1, SOD or Hb prevented DNA from fragmentation.
(2) Cell Viability: Trypan blue stained cells, whose membrane integrity has been lost, are necrotic cells or late-phase apoptotic cells. The number of dead cells increased gradually 8 hrs after the exposure to NO donors (See Figure 1: the data presented as the mean ± SD derived from 3 experiments). In contrast, EPC-K1, SOD and Hb effectively protected the cells from death (See Figure 2: the data presented as the mean ± SD derived from 3 experiments. In the figure, "a" indicates p<0.01 as compared with the normal cells, "b" <0.05 as compared with 100 µmol/L SNP-treated cells, and "c" <0.05 as compared with 100 µmol/L GSNO-treated cells, respectively).
(3) Mitochondrial Activity: The tetrazolium salt, MTT, can be reduced to a formazan by mitochondrial respiratory enzymes.. The amount of the formazan thus formed serves as an accurate indicator of the number of viable cells and the mitochondrial activity [Mosmann, T., (1983), J. Immunol. Methods, 65, 55-63; Hansen, M. B., Nielsen, S. E., and Berg, K., (1989), J. Immunol. Methods, 119, 203-210]. Figure 3 (the data presented as the mean ± SD derived from 6 experiments) shows that the ability of cells to reduce MTT decreased significantly after the 8-hr exposure to 100 µmol/L GSNO. This was due to inactivation of mitochondrial enzymes, and not to the loss of viable cells, for the decrease in the number of viable cells, as compared with the normal cultures, was only negligible. While pre-treatment of the cells with EPC-K1 or SOD exhibited no dear protective effects, they surely prevented mitochondrial enzymes from falling into further dysfunction On the other hand, Hb effectively protected mitochondrial enzymes from NO donors-induced inactivation.
(4) Lipid Peroxidation in Cells: The 24-hr treatment of the cells with GSNO or SNP significantly increased the formation of TBARS, indicating that it initiated lipid peroxidation. Pretreatment with EPC-K1 or SOD effectively inhibited lipid peroxidation of the membrane (See Figure 4: the data presented as the mean ± SD derived from 3 experiments. "a" indicates p<0.01 as compared with the normal cells, "b" <0.05 as compared with the normal cells, "c" <0.05 as compared with 100 µmol/L SNP-treated cells, "d" <0.05 as compared with 100 µmol/L GSNO-treated cells).
(5) Peroxynitrite Induced Cell Death: Apoptosis was found to take place after a 30-min exposure to 5-10 µmol/L peroxynitrite. Pretreatment of the cells with EPC-K1 effectively prevented cell death (See Figure 5: assessment made of cell viability based on trypan blue exclusion and MTT assay after 24-hr culture).

### C. Discussion:

NO, which is a highly reactive free radical, acts as both a physiological messenger and a neurotoxic agent in mammalian central nervous systemss [Gross, S. S., and Wolin, M. S.,(1995), Annu. Rev. Physiol., 57, 737-769]. While recent studies have revealed that NO induces apoptosis in several kinds of neurons *in vitro*, while the exact mechanisms are still not clear. Among the pathways involved in NO-mediated neurotoxicity, the relationships and interactions between NO and excitatory amino acid receptors (e.g., NMDA-R) has drawn much attention [Lipton, S. A., Choi, Y. B., Pan, Z. H., Lei, S. Z., Chen, H. V., Sucher, N. J., Loscalzo, J., Singel. D. J., and Stamler, J. S.,(1993), Nature, 364, 626-632]. On the one hand, excitotoxicity triggered by NMDA-R over activation requires the synthesis and involvement of NO [Dawson, V. L., Dawson, T. M., London, E. D., Bredt, D. S., and Snyder, S. H.,(1991), Proc. Natl. Acad. Sci. USA, 88, 6368-6371]. On the other hand, NO induces the release of NMDA agonists and subsequently activates NMDA-R channels, and thus triggers a series of physiological and biochemical signals including Ca²⁺ influx [Bonfoco, E., Leist, M., Zhivotovsky, B., Orrenius, S., Lipton, S. A., and Nicotera, P.,(1996), J. Neurochem., 67, 2484-2493].

Actually, many other factors, such as reactive oxygen species, may also play important roles in the cascade leading to NO-induced apoptosis. The objective of the present investigation was to examine whether ROS are relevant to NO-induced neuronal toxicity. In order to exclude the involvement of excitatory amino acid receptors, immature cultures of rat cerebellar granule cells (2 days *in* *vitro*) were employed in the experiment. This type of culture consists of relatively homogenous neurons (> 90 %), to which excitatory amino adds only trigger slight neurotoxicity [Resink, A. Hack, N., Boer, G. J., and Balazs, R.,(1994), Brain Res., 655, 222-232].

Morphological and biochemical evidences both indicated that exposure to NO donors induces apoptosis in immature cultures of cerebellar granule cells. After treating neurons with GSNO or SNP for 24 hrs, condensed chromatin and fragmented nuclei were seen by fluoromicroscopy and electromicroscopy. Agarose gel electrophoresis of DNA extracted from the neurons treated with the NO donors shows a ladder patter, which is regarded as a biochemical characteristic of apoptosis. Breakdown products of the NO donors had no influence on the cell viability, while Hb, which is a specific scavenger of NO, protected neurons from injury. These suggest tat the neurotoxicity of NO donors is based on the release of NO. Unlike the previous report [Bonfoco, E.; Leist, M., Zhivotovsky, B., Orrenius, S., Lipton, S. A., and Nicotera, P.,(1996), J. Neurochem., 67, 2484-2493] this induction of apoptosis by NO donors did not require the activation of NMDA-R.

Our results showed that the ability of cells to reduce MTT (a marker of mitochondrial function) significantly decreased after a 8-hr exposure to GSNO. Hb effectively protected the mitochondrial function, suggesting that it was NO that caused the dysfunction of mitochondria. NO can diffuse across the plasma membrane as well as mitochondrial membrane, and react with thiol groups, iron-sulfur dusters and heme proteins, and thus influence the activity of enzymes [Stamler, J. S.,(1994), Cell 78, 931-936] including complex I, complex II-III and complex IV of the mitochondrial electron transport chain [Bolanos, J. p., Heales, S. J. R., Peuchen, S., Barker, J. E., Land, J. M., and Clark, J. B.,(1996), Free Radic. Biol. Med., 21, 995-1001]. Inactivation of mitochondrial enzymes leads to the lowering of ATP level, which may play an important role in NO-induced cell apoptosis [Richter, C., Schweizer, M., Cossarizza, A., and Francesmi, C.,(1996), FEBS Lett., 378, 107-110].

Pretreatment of the cells with EPC-K1 or SOD prevented mitochondria from receiving further damages, indicating that reactive oxygen species, especially superoxide anion and its breakdown products such as peroxynitrite, might play important roles in NO-induced mitochondrial dysfunction. However, blockage of mitochondrial respiration may cause the formation of superoxide anion [Poderoso, J. J., Carreras, M. C., Lisdero, C., Riobo, N., Schopfer, F., and Boveris, A.,(1996), Arch. Biochem. Biophys., 328, 85-92], which can react with NO at very high rates to form peroxynitrite, a potent oxidant. Peroxynitrite may oxidize cysteine and tyrosine residues of enzymes and thereby influence their activities. In addition, peroxynitrite decomposes at physiological pH into highly reactive species such as hydroxy radicals, which may damage the biomacromolecules, causing a series of free radical-associated injuries. Our results also demonstrated that exposure of cells to peroxynitrite leads to mitochondrial dysfunction and cell death. Taken together, NO inhibited the respiratory chain and thereby induced the formation of reactive oxygen species such as peroxynitrite, which then caused more serious damages to mitochondria [Bolanos, J. P., Heales, S. J. R., Land, J. M., and Clark, J. B.,(1995), J. Neurochem., 64, 1965-1972], and cell death was resulted finally. In order to study whether other downstream products of superoxide (mainly, hydrogen peroxide and its decomposed product hydroxyl radical) were involved in the oxidative injuries initiated by NO, the effect of exogenous catalase on cells was examined. Pretreatment of cells with catalase (100 U/mL) had no protective effect against NO-induced apoptosis (data not shown). This suggests that peroxynitrite and/or its breakdown products were main mediators of NO-induced oxidative injuries.

The number of trypan blue-stained cells increased time-dependently 8 hrs after the addition of NO donors. This might be due to a secondary necrosis after the activation of the apoptotic program. Treatment of cells with free radical scavengers significantly increased the number of cells which excluded the dye, indicating that it served to preserve the integrity of the cell membrane. The results of TBA assay showed that NO initiated lipid peroxidation in neuronal cells *in vitro*, which caused severe damage to the cell membranes. EPC-K1 and SOD effectively inhibited lipid peroxidation and thus protected the membrane from oxidative injury, which results were in consistent with the results of trypan blue assay. On the other hand, unpublished data by our laboratory [Taotao Wei et al., contributed to Free Radic. Biol. Med.] demonstrated that EPC-K1 is an effective scavenger of peroxynitrite and protects neuronal membrane fluidity from oxidative stress-induced alteration. These results suggests that the protective effects of EPC-K1 and SOD against NO/ONOO⁻-induced apoptosis are, at least partially, due to the protection of cell membrane.

In conclusion, the above results indicates that free radical scavengers such as EPC-K1 can attenuate NO-mediated cytotoxicity by eliminating the highly reactive downstream products of NO, ONOO⁻. On the other hand, NO also inactivates glutathione peroxidase [Asahi, M., Fujii, J., Suzuki, K., Seo, H. G., Kuzuya, T., Hori, M., Tada, M., Fujii, S., and Taniguchi, N., (1995), J. Biol. Chem., 270, 21035-21039] and damages the cellular antioxidant defense systems, thereby causing oxidative stress. With this regard, exogenous antioxidants can enhance the cellular antioxidant ability, thereby protecting the cells.

### 〈Pharmacological Test 2〉

In an experiment of peroxynitrite-induced neuronal degeneration, the effects of EPC-K1, vitamin C and Trolox were compared, further employing the influence on ATP content and membrane fluidity as additional measures.

### A. Materials and Methods:

(1) Materials: Culture dishes purchased from Costar (Cambridge, MA, USA) were used. Fetal bovine serum and ribonuclease A (RNase A) were purchased from Gibco BRL (Grand Island, NY, USA). Agarose was purchased from Sigma (St. Louis, MO, USA). 6-Hydroxy-2,5,7,8-tetramethylchroman-2-carboxylic acid (Trolox) was purchased from Aldrich (Milwaukee, WI, USA). The route for purchase of animals and other materials were the same as in Pharmacological Test 1. Peroxynitrite was synthesized in a quenched flow reactor [Zhao, B.L., Shen, J.G., Li, M., Li, M.F., Wan, Q., and Xin, W.J., (1996), Biochim. Biophys. Acta, 1315, 131-137] and diluted with 10 mM/L NaOH just before use. Other reagents made in China were of analytical grade.
(2) Cell Culture: Cerebella of 7-day-old Wistar rats were collected, rinsed with HBSS, and dissociated by mild trypsinization. The cells were plated in 6-well multidishes (2 × 10⁶ cells/mL, 2mL/well) or 24-well multidishes (2.5 × 10⁶ cells/mL, 0.4 ml/well) previously coated with poly-L-lysine. Culture media used was DMEM supplemented with KCL (19.6 mmol/L), glutamine (2 mmol/L), HEPES (10 mmol/L), and fetal calf serum (10 %, v/v); The cells were maintained at 37°C in a humidified 5 % CO₂-95 % air atmosphere. Experiments were carried out 48 hrs after plating.
(3) Treatment with peroxynitrite: The culture media were removed, cells were washed twice with HBSS, and incubated in HBSS containing 25 mM HEPES (pH 7.4) at 37°C for 15 min. Antioxidants (EPC-K1, vitamin C, Trolox), when employed, were added during this period. Aliquots of peroxynitrite stock solution were rapidly added to the wells and gently mixed. Control cells received the same volume of 10 mM NaOH solution. After incubation with peroxynitrite at 37°C for 30 min, the cells were washed twice with HBSS, and cultured with the original culture medium restored. Under these experimental conditions, the addition of peroxynitrite caused only slight pH shifts.
(4) Morphological Studies: The cells were observed using a phase contrast microscope, a fluoromicroscope and an electron microscope following the procedures described in Pharmacological Test 1.
(5) Detection of DNA Fragmentation: The laddering pattern of DNA fragmentation was detected by agarose gel electrophoresis according to a previous report [Didier, M., Bursztajn, S. et al., J. Neurosci., 16: 2238-2250 (1996)]. Briefly, the cells (1.2 × 10⁷) were pelletted, rinsed with cold PBS, and lysed in 10 mM Tris, 10 mM EDTA, 0.2 % Triton X-100, pH 7.5. After 15 min on ice, the lysate was centrifuged at 12,000 × g for 10 min. The supernate (which contained RNA and fragmented DNA but not intact chromatin) was treated with 0.5 mg/mL proteinase K and 0.5 mg/mL RNase A at 50°C for 3 hrs. After phenol extraction and then chloroform extraction, DNA was participated with 0.1 volume of 10 M ammonium acetate and 2 volumes of ethanol at -20°C, left stand overnight, and harvested by centrifugation at 12,000×g for 15 min. After washing with 70% ethanol, DNA was dissolved in TE (10 mM Tris, 1 mM EDTA, pH 8.0) and analyzed by 1.5 % agarose gel electrophoresis.
(6) Assessment of Cell Viability: Cell viability was assessed by MTT assay. MTT was added to cells cultured in 24-well multidishes (0.5 mg/mL, final concentration). After 30-min incubation at 37°C, 1 mL of lysis solution (10 % SDS, 25 % DMF, pH 3.5) was added and optical density was measured at 570 nm.
(7) Measurement of Lipid Peroxidation: Lipid peroxidation of cerebellar granule cells was measured by thiobarbituric acid (TBA) assay.
(8) Spin Labeling: Cell membrane fluidity was determined by ESR spin labeling technique. Cells (1.2× 10⁷) were incubated with 100 µM 5-doxyl- stearic acid in PBS at 37°C for 30 min, washed 3 times with PBS, and transferred into quartz capillaries for ESR measurement. ESR spectrum was recorded on a Varian E-109 spectrometer with measurement conditions of: X-band, 100 kHz modulation with 0.1 mT modulation amplitudes, 10 mW microwave power, 25°C. The order parameter (S) was calculated from the ESR spectrum according to the description in Zhao B.L. et al., Kexue Tongbao, 28, 392-396 (1983).
(9) Measurement of ATP: Cellular ATP content was measured by HPLC. Briefly, 1.2× 10⁷ cells were pelletted and treated with 0.5 M phosphoric acid for 30 min on ice. After centrifugation at 12,000×g for 10 min, the supernate was neutralized and then analyzed by HPLC (Zorbax 5 µm ODS column, 250×4.6 mm). The eluant was 0.05 M phosphate buffer containing 3.75 % methanol (pH 6.0).
(10) Statistical Analyses: Each experiment was performed at least three times and the results were presented as mean ± SD. Statistical analyses were performed using one-way ANOVA or Student's t-test. A probability of <0.05 was deemed significant.

### B. Test Results:

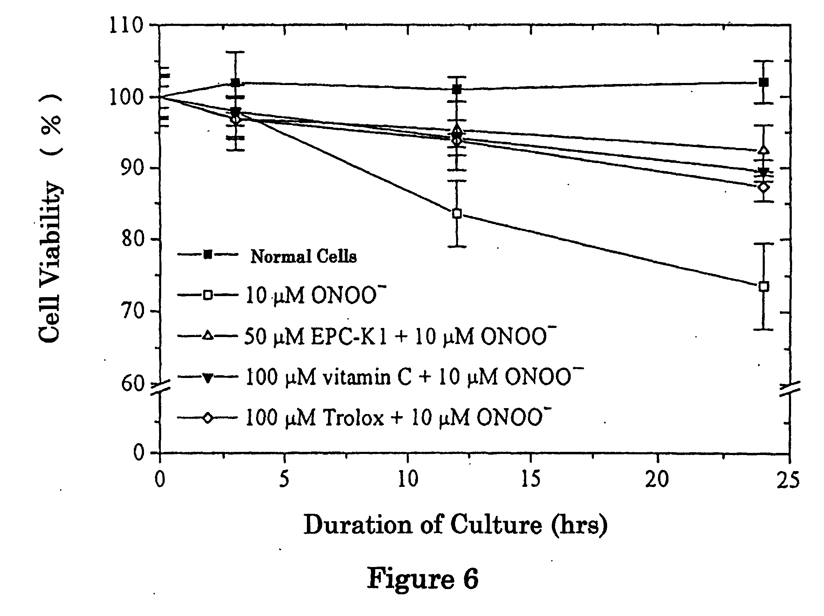

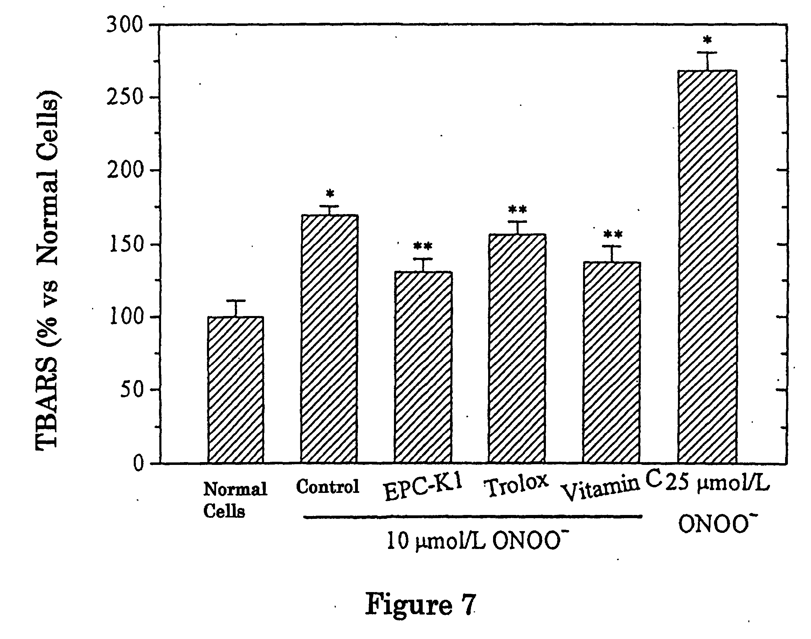

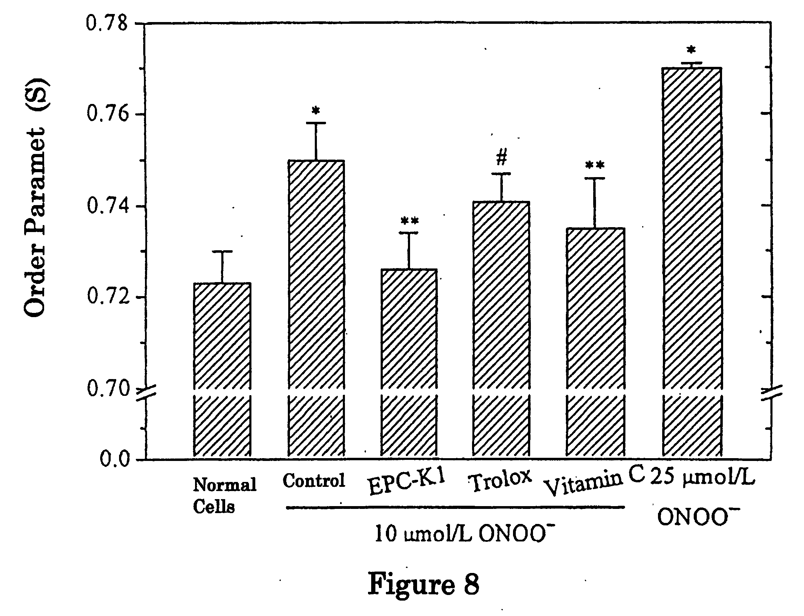

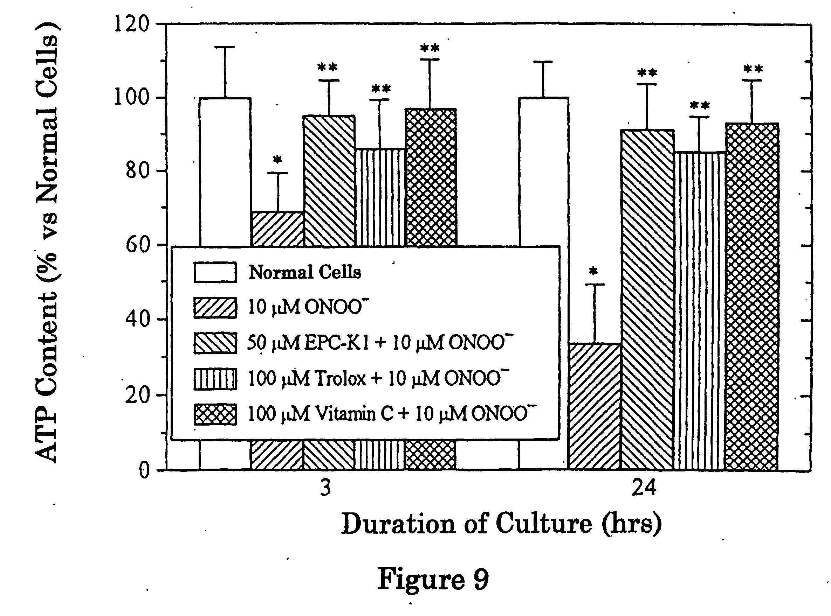

(1) Characteristics of Apoptosis: After a 30-min exposure to 10 µM peroxynitrite, cerebellar granule cells underwent apoptosis, which was morphologically characterized by neurite breakdown, cell shrinkage, chromatin condensation, and the formation of apoptotic bodies. Cells pretreated with antioxidants EPC-K1, vitamin C or Trolox (100 µM) were protected from death. Agarose gel electrophoresis of DNA extracted from peroxynitrite-treated cells showed a ladder pattern characteristic of apoptosis. Pretreatment of cells with 50 µM EPC-K1 effectively prevented DNA from fragmentation. Vitamin C and Trolox also exhibited protective effects at a higher concentration (100 µM).
(2) Cell Viability: The number of dead cells increased gradually after exposure to peroxynitrite as assessed by MTT assay. EPC-K1 effectively protected the cells from death (Figure 6). Vitamin C and Trolox exhibited similar protective effects at a higher concentration (100 µM).
(3) Lipid Peroxidation in Cells: As indicated by the significant increase in TBARS levels, exposure of the cultures to peroxynitrite caused lipid peroxidation in the cells. In cultures pretreated with 50 µM EPC-K1, the formation of TBARS was markedly suppressed, suggesting that EPC-K1 effectively inhibited membrane lipid peroxidation (Figure 7). Pretreatment of cells with 100 µM vitamin C or Trolox also markedly inhibited the formation of TBARS.
(4) Cell Membrane fluidity Alteration: Order parameter calculated from 5-doxyl spin label ESR spectrum serves as an index of the membrane fluidity. After exposure to peroxynitrite, cell membrane fluidity decreased significantly, and antioxidants EPC-K1 (50 µM) or vitamin C (100 µM) prevented the change in cell membrane fluidity, but Trolox did not show significant protective effect even at a concentration of 100 µM (Figure 8).
(5) ATP Content: The cellular ATP content decreased time-dependently after exposure to peroxynitrite for 30 min (Figure 9). However, pretreatment of the cells with antioxidant EPC-K1, vitamin C or Trolox suppressed the decrease of ATP.

### C. Discussion:

NO is generated endogenously by several kinds of neuronal cells including cortical neurons, cerebellar granule cells, and astrocytes. This means that peroxynitrite may be formed in the central nervous system under some pathological conditions. Peroxynitrite (and its breakdown product, hydroxy radical) can oxidize biomacromolecules including membrane, protein and DNA, and cause oxidative injuries. In the experiment, ONOO⁻-induced neuronal cell injury was employed as a model of NO/ONOO⁻ -initiated neurotoxicity. The results of the experiment indicate that treatment of cerebellar granule cells with peroxynitrite induces apoptosis and antioxidant EPC-K1 has inhibitory effect against this process. Peroxynitrite-induced lipid peroxidation in cerebellar granule cells damaged the normal structure and function of the neuronal cell membrane. Oxidative stress may alter the permeability of membrane and thereby trigger series of signals such as Ca²⁺ influx, which generates different signals leading to further production of NO. Furthermore, peroxynitrite may oxidize cysteine and tyrosine residues of the complexes of the mitochondrial electron transport chain and thereby influence their activity, which possibility is supported by the decrease in ATP content confirmed in the above experiment. Decrease in ATP content may trigger a series of signals leading to apoptosis, and suppression of this decrease therefore is considered to be an important factor effectively serving to block apoptosis. EPC-K1 exhibited protective action more effective than vitamin C or Trolox against ONOO⁻-induced apoptosis. Considering practical application, EPC-K1 allows quick administration into the circulation by injection for it is soluble in water, and it also has an advantage, when being transferred to brain tissues through the wall of blood vessels, in that it has a large hydrophobic moiety in its molecule.

The present invention is described in further detail below with reference to representative examples. It is not intended, however, that the present invention be limited to the examples.

### 〈Example 1〉 Oral Tablets

The following components shown for one tablet are mixed by a conventional method and compressed into tablets by a conventional method.

| | |
|---|---|
| EPC-K1 | 100 mg |
| Lactose | 75 mg |
| Corn starch | 20 mg |
| Polyethylene glycol 6000 | 5 mg |
| Total amount | 200 mg |

### 〈Example 2〉 Oral Tablets

The following components shown for one tablet are mixed by a conventional method and compressed into tablets by a conventional method.

| | |
|---|---|
| EPC-K1 | 50 mg |
| Corn starch | 90 mg |
| Lactose | 30 mg |
| Hydroxypropylcellulose | 25 mg |
| Magnesium stearate | 5 mg |
| Total amount | 200 mg |

### 〈Example 3〉 Injections

The following components are mixed to dissolve, sterilized by filtration and the filtrate aseptically filled into aseptic glass ampoules, which then are fused to seal to prepare injections.

| | |
|---|---|
| EPC-K1 | 200 mg |
| Mannitol | 5.0 g |
| 1N sodium hydroxide | q.s. |
| Distilled water for injection | q.s. |
| Total amount | 100 mL |

### 〈Example 4〉 Injections

The following components are mixed to dissolve, sterilized by filtration and the filtrate aseptically filled into aseptic glass ampoules, which then are fused to seal to prepare injections.

| | |
|---|---|
| EPC-K1 | 20 mg |
| Glucose | 5.0 g |
| 1N NaOH or HCl | q.s. |
| Distilled water for injection | q.s. |
| Total amount | 100 mL |

### Industrial Applicability

The present invention can provide safe medicaments for prevention and treatment of a variety of diseases accompanied by neuronal apoptosis including Alzheimer's disease, Parkinson's disease, amyotrophic lateral sclerosis and the like.

## Claims

1. A medicament for prevention and treatment of a neurodegenerative disease comprising a compound represented by the formula, 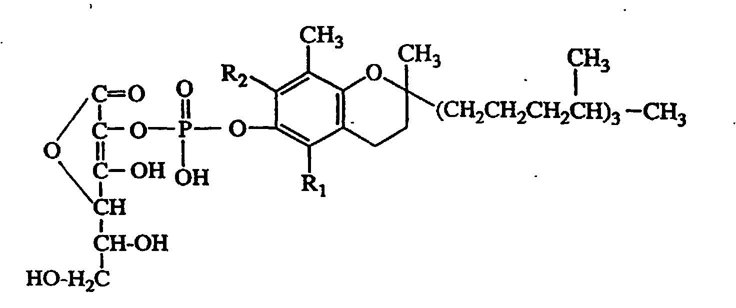 wherein R1 and R2 are the same or different and each denotes hydrogen or methyl, or a pharmaceutically acceptable salt thereof.

2. The medicament for prevention and treatment of a neurodegenerative disease of claim 1 wherein said neurodegenerative disease is Alzheimer's disease, Parkinson's disease or amyotrophic lateral sclerosis.

3. The medicament for prevention and treatment of a neurodegenerative disease of claim 1 or 2 in the form of an injection or a solid preparation for oral administration.

4. A method for prevention and treatment of a neurodegenerative disease in a mammal including a human comprising administering to the mammal including a human an effective amount of a compound represented by the formula, 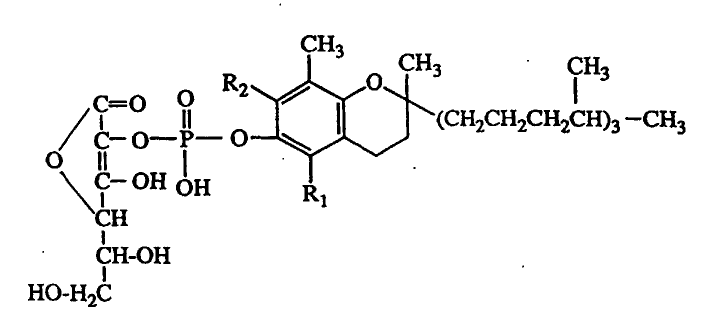 wherein R1 and R2 are the same or different and each denotes hydrogen or methyl, or a pharmaceutically acceptable salt thereof.

5. The method for prevention and treatment of a neurodegenerative disease of claim 4 wherein said neurodegenerative disease is Alzheimer's disease, Parkinson's disease or amyotrophic lateral sclerosis.

6. Use of a compound represented by the formula, 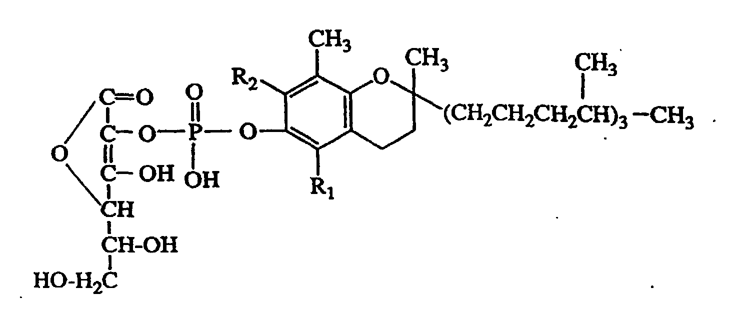 wherein R1 and R2 are the same or different and each denotes hydrogen or methyl, or a pharmaceutically acceptable salt thereof, for preparation of a medicament for prevention and treatment of a neurodegenerative disease in a mammal including a human.

7. The use of claim 6 wherein said neurodegenerative disease is Alzheimer's disease, Parkinson's disease or amyotrophic lateral sclerosis.

8. The use of claim 6 wherein said medicament is in the form of an injection or a solid preparation for oral administration.
